# EUROPÄISCHE PATENTSCHRIFT

(11) **EP 2 331 260 B1**
(45) Veröffentlichungstag und Bekanntmachung des Hinweises auf die Patenterteilung: **04.10.2017**
(21) Anmeldenummer: 09775596.1
(22) Anmeldetag: 14.08.2009
(51) Int. Cl.: B01L 3/00, B01L 3/14, B01L 7/00, B01F 7/00, G01N 33/543

(54) **VORRICHTUNG ZUM TEMPERIEREN EINES ROTATIONSSYMMETRISCHEN BEHÄLTNISSES**
DEVICE FOR THERMALLY REGULATING A ROTATIONALLY SYMMETRICAL CONTAINER
DISPOSITIF POUR PORTER À L'ÉQUILIBRE DE TEMPÉRATURE UN RÉCIPIENT À SYMÉTRIE DE ROTATION

(30) Priorität: 29.08.2008 AT 13502008
(43) Veröffentlichungstag der Anmeldung: 15.06.2011
(73) Patentinhaber: Cube Dx GmbH, 4300 St. Valentin (AT)
(72) Erfinder: RONACHER, Bernhard, A-4020 Linz (AT)
(74) Vertreter: KLIMENT & HENHAPEL
(86) Internationale Anmeldenummer: PCT/AT2009/000312
(87) Internationale Veröffentlichungsnummer: WO 2010/022417

(56) Entgegenhaltungen:
- WO-A-03/000428
- WO-A-03/100401
- WO-A-2007/041734
- DE-A1- 19 939 252

## Beschreibung

Die vorliegende Erfindung betrifft eine Vorrichtung zum Temperieren eines rotationssymmetrischen Behältnisses.

Das stufenweise Temperieren insbesondere das abwechselnde Heizen und Kühlen von Flüssigkeiten zur Steuerung von biochemischen Nachweisreaktionen erhielt Bedeutung durch die Kombination von Primern und dem Einsatz von thermostabilen DNA Polymerasen bei der Polymerasenkettenreaktion (PCR), sowie der Ausbildung von Nukeleinsäuredoppelsträngen im Zuge von Hybridisierungsereignissen (Southern Hybridisierung). Nach der Entwicklung der PCR kamen noch andere Enzyme, wie Ligasen und Kinasen usw., dazu. Allen gemeinsam ist das Prinzip, dass die Flüssigkeit und die darin enthaltenen Bestandteile (Reaktionspartner) temperiert werden und die Flüssigkeit eine Reaktionseinheit darstellt (einheitliche Temperatur und Zusammensetzung haben).

Derartige Reaktionen können jedoch nicht nur rein in Flüssigkeit erfolgen, sondern können auch feste Phasen umfassen, wobei diese zur Durchführung von Festphasenreaktionen eingesetzt werden können. Dabei wird zumindest ein Reaktionspartner auf einer festen Phase immobilisiert. In der einfachsten Form können "solides" (meist Objektträger aus Glas) als feste Phasen/Träger eingesetzt werden, die auf einen Metallblock gelegt und mit einer Flüssigphase überschichtet werden. Die Verdunstung kann dabei durch eine Haube (Kammer) minimiert werden. Dadurch entsteht wenigstens für die Reaktionsdauer ein abgeschlossener Raum. Die Reaktionspartner, wie z.B. Primer bei einer PCR-Reaktion, können dabei an die Slideoberfläche kovalent immobilisiert werden und stellen die Ausgangspunkte für die Nachweisreaktion dar. Gemeinsam ist diesen Systemen, dass die mobile Phase wieder als Einheit gesehen werden kann und in der Zusammensetzung im Wesentlichen einheitlich ist.

Als drittes System sind die thermostatisierten Microfluidik Systeme zu sehen. Dabei wir die mobile Phase (mit allen Reaktionspartnern) durch eine vordefinierte Bahn (Kanal, Kapillare) gepumpt. Dabei durchläuft die mobile Phase Zonen, in denen die Wände (Umgrenzung) unterschiedliche Temperaturen (die konstant sein können) haben. Vorteil dieser Systeme ist, dass der Temperaturwechsel in der mobilen Phase rasch erfolgen kann (s. z.B. WO 00/23190, WO 01/89692).

In der US 5,433,080, JP 2000005639, JP 2001153487 und der EP 1 845 372 werden Zentrifugen beschrieben, welche beheizbar sind.

In der US 4,531,932 wird eine Plasmapherese-Vorrichtung beschrieben, die ein Behältnis aufweist, in dem ein rotations symmetrischer Rotor angeordnet ist. Um das Kühlen des in dieser Vorrichtung befindlichen Blutes zu verhindern, weist der Rotor in der Mitte Temperierelemente auf.

Im Stand der Technik sind zahlreiche Verfahren und Vorrichtungen zum Temperieren von Flüssigkeiten in der Analytik, insbesondere für PCR-Verfahren beschrieben, bei denen insbesondere zwei Bereiche von hoher Bedeutung sind:
a) Geschwindigkeit und Genauigkeit der Heiz- und Kühlraten
b) Anzahl der unterschiedlichen Reaktionen
Die schnellsten, noch exakten Heiz- und Kühlraten, die derzeit mit Temperierblöcken aus Metall im Bereich der Nukleinsäureamplifikationen (PCR) erzielt werden, liegen bei 5-7°C pro Sekunde. Dabei kommt insbesondere der Genauigkeit der zu erreichenden Temperaturen besondere Aufmerksamkeit zu. Denn die Annealing-Temperatur oder Schmelztemperatur von DNA darf nicht wesentlich über- bzw. unterschritten werden, da es ansonsten zu unerwünschten Nebenreaktionen kommen kann. Dies bedeutet, dass eine sehr langsame Annäherung an die Zieltemperatur erfolgt. Dadurch verlängern sich sowohl die Heiz- als auch Kühlzeiten erheblich, wobei betreffend Genauigkeit auch noch die Menge und Zusammensetzung der mobilen Phase in Betracht zu ziehen ist. Bei den Microfluidik-Systemen, bei denen sich sämtliche Reaktionspartner in Lösung befinden und in, beispielsweise, Kapillaren transportiert werden, wurde dieses Problem dadurch gelöst, dass die Temperaturzonen, durch die die Kapillaren geführt werden, konstante Temperaturen aufweisen. Nachteil dieser Systeme ist jedoch, dass alle Reaktionspartner in der mobilen Phase mitgeführt werden müssen. Eine Kombination mit einem Festphasenreaktionssystem ist dabei nicht möglich.

Vor allem bei den löslichen Primern (PCR) ist bekannt, dass es beim gleichzeitigen (parallelen) Einsatz von vielen verschiedenen Primern zu unerwünschten Reaktionen kommen kann. Dies führt dann zu Signalen, die zu falsch-positiven bzw. falsch-negativen Ergebnissen führen. Dadurch sind Multiplex-Systeme (mehrfache Nachweisreaktionen in einem einzigen Test) vor allem im Bereich des Nachweises von Nukleinsäuren in der Praxis nur bedingt brauchbar. Einen Ausweg bietet das Aufteilen der Proben in mehrere Aliquote, die dann wiederum in Einzeltests analysiert werden können. Das Aufteilen von geringen Probenmengen unterliegt großen unvermeidbaren statistischen Fehlern einerseits und treibt andererseits den Zeit und Kostenaufwand in die Höhe.

Es ist somit ein Ziel der vorliegenden Erfindung, eine Vorrichtung zur Verfügung zu stellen, die es ermöglicht, in einem einzigen Behältnis unterschiedliche Temperaturbereiche zu schaffen, um beispielsweise eine Vielzahl an unterschiedlichen Nachweisreaktionen parallel in einem einzigen oder in einigen wenigen Testsystemen durchzuführen. Weiters ist es ein Ziel, eine Vorrichtung bereitzustellen die schnelle und genaue Heiz- und Kühlzyklen ermöglicht. Ein weiteres Ziel ist es dabei auch, dass immobilisierte Reaktionspartner (Primer) in diesem einzelnen Behältnis abwechselnd in die unterschiedlichen Temperaturzonen geführt werden.

Die vorliegende Erfindung betrifft eine Vorrichtung zum Temperieren eines rotationssymmetrischen Behältnisses mit einer Mantelfläche und/oder einer Grundfläche umfassend mindestens einen zur Aufnahme des Behältnisses geeigneten Temperierblock mit mindestens zwei Temperierelementen, wobei die Temperierelemente des mindestens einen Temperierblocks mit der Mantelfläche und/oder mit der Grundfläche des zu temperierenden Behältnisses in Wärmeaustauschkontakt gebracht werden können.

Durch das Vorsehen eines Temperierblocks mit mehr als einem Temperierelement (mindestens zwei, vorzugsweise mindestens drei, vier, fünf, sechs, sieben, acht, neun oder zehn) ist es mit der erfindungsgemäßen Vorrichtung möglich, in einem Behältnis, welches in Wärmeaustauschkontakt mit dem Temperierblock bringbar ist, verschiedene Temperaturbereiche zu schaffen. Durch das Vorsehen unterschiedlicher Temperaturbereiche können innerhalb des Behältnisses unterschiedliche Temperaturbedingungen geschaffen werden. Dies ist beispielsweise von Vorteil, wenn an der Mantelinnenfläche unterschiedliche Enzyme immobilisiert sind, die bei verschiedenen Temperatur Reaktionsoptima aufweisen. Auf diese Weise können im Behältnis Reaktionen mit unterschiedlichen Reaktionsbedingungen parallel durchgeführt werden.

Das Behältnis, welches in den Temperierblock aufgenommen werden kann, kann im Temperierblock rotatorisch (radial) fixiert oder rotatorisch (radial) nicht fixiert sein. Ist das eingebrachte Behältnis rotatorisch fixiert, ist es nicht in der Lage innerhalb des Temperierblocks zu rotieren.

Der Temperierblock weist eine Form auf, die geeignet ist, ein rotationssymmetrisches Behältnis aufzunehmen, und zwar in der Weise, dass die im Temperierblock vorgesehenen Temperierelemente zumindest mit der Grundfläche oder der Mantelfläche des Behältnisses in Wärmeaustauschkontakt bringbar sind. Daher ist es bevorzugt, dass der Temperierblock eine dem Behältnis äquivalente Form aufweist. Ein Wärmeaustauschkontakt zwischen den Temperierelementen und dem Behältnis kann dadurch hergestellt werden, dass sich beide Elemente direkt berühren, oder dass sich zwischen der Mantelfläche/Grundfläche des Behältnisses und der Temperierelemente Mittel zur Übertragung von Wärme befinden (z.B. wärmeleitende Mittel).

Um das zu temperierende Behältnis in den Temperierblock einzubringen, ist das Behältnis vorzugsweise translatorisch relativ zum mindestens einen Temperierblock bewegbar angeordnet. Dadurch wird es ermöglicht, das Behältnis nach dessen Verwendung auch aus dem Temperierblock zu entnehmen. Gegebenenfalls kann der Temperierblock eine Arretierungsvorrichtung aufweisen, die eine translatorische und/oder radiale Bewegung des Behältnisses relativ zum Temperierblock verhindert. Dadurch wird verhindert, dass bei Verwendung der Vorrichtung das Behältnis relativ zum Temperaturblock unkontrolliert bewegt wird, was zu nicht gewünschten Temperaturunterschieden führen kann. Vorzugsweise ist daher im Betrieb das Behältnis im Wärmeaustauschkontakt mit den Temperierelementen relativ zum Temperierblock fixiert. So kann erreicht werden, dass sobald das Behältnis in Wärmeaustauschkontakt gebracht wird, dieses translatorisch fixiert werden kann.

Mit der erfindungsgemäßen Vorrichtung können verschiedene Verfahren bzw. Verfahrensschritte durchgeführt werden. So eignet sich die Vorrichtung zum Heizen und Kühlen von Flüssigkeiten zum Zwecke der gezielten Ausführung von biochemischen Reaktionen (z.B. Denaturieren von DNA, PCR, Ligase Reaktionen, allgemeine Enzym Reaktionen...).

Das Behältnis kann jegliche Form aufweisen sofern diese rotationssymmetrisch (z.B. Zylinder, Kegel, Kegelstumpf) ist. Dadurch wird es ermöglicht, gegebenenfalls das Behältnis im Temperierblock rotatorisch zu bewegen, ohne den Wärmeaustauschkontakt zwischen Behältnis und Temperierblock zu unterbrechen. Ferner ist es durch diese Form auch möglich, in ein rotatorisch fixiertes Behältnis einen Rotor einzubringen, der innerhalb des Behältnisses rotatorisch bewegt werden kann, und die Mantelfläche dieses Rotors mit der Innenmantelfläche des Behältnisses einen Ringspalt ausbildet, damit der Abstand der Mantelfläche des Rotors zur Mantelinnenfläche des Behältnisses während der Rotation im Wesentlichen konstant bleibt. Vorzugsweise weist das rotationssymmetrische Behältnis die Form eines Zylinders auf, da bei dieser Form der Weg jedes Punktes an der Mantelfläche des Behältnisses in einer Rotationsbewegung gleich ist. Dadurch wird garantiert, dass das Behältnis gleichermaßen über die gesamte Länge temperiert wird.

Entsprechend der Form des Behältnisses sind auch die Temperierelemente geformt. So können diese beispielsweise die Form von Backen aufweisen. Oder die Form von Stäben, Rechtecken, Kreisflächen (elliptisch), sowie Drähte oder Fasern aufweisen.

Die Art des Wärmetransports kann über Berührungsflächen (bevorzugt Metallflächen) erfolgen. Oder durch berührungslosen Wärmeaustausch erfolgen, wie z.B. Strahlungsenergie (Infrarot, Mikrowelle), temperierte Gase oder Flüssigkeiten, Reibung, elektrische Ladungsaustausch...).

Das Behältnis ist zur Analyse von gasförmigen und/oder flüssigen Proben geeignet und weist einen in das Behältnis einsetzbaren rotationssymmetrischen Rotor auf, wobei zwischen dem Behältnis und dem Rotor ein Ringspalt vorgesehen ist und der Rotor mindestens einen Strömungskanal zur Beförderung von Flüssigkeiten und/oder Gasen in und/oder aus dem Innenraum des Behältnisses aufweist.

Vorrichtungen zur Analyse von flüssigen Proben, die einen darin befindlichen Rotor aufweisen, sind im Stand der Technik bereits bekannt (siehe z.B. WO 2007/041734 und WO 03/100401). Diese Behältnisse eignen sich besonders gut zur Verwendung in einer erfindungsgemäßen Vorrichtung.

Gemäß einer bevorzugten Ausführungsform der vorliegenden Erfindung sind an der Oberfläche des Rotors Biomoleküle immobilisiert. Durch das Vorsehen von Biomolekülen an der Oberfläche des Rotors können diese im Zuge der Rotation durch das Medium innerhalb des Behältnisses bewegt werden. Dadurch können die an der Oberfläche immobilisierten Biomoleküle im Zuge der Rotation unterschiedlichen Temperaturen ausgesetzt werden, je nachdem welche Temperierbereiche passiert werden.

Alternativ dazu sind an der den Temperierelementen gegenüberliegenden Oberfläche der Grundfläche des Behältnisses vorzugsweise Biomoleküle immobilisiert.

In einer weiteren Ausführungsform der vorliegenden Erfindung können an der Grundfläche des Behältnisses die Biomoleküle immobilisiert werden.

Die erfindungsgemäße Vorrichtung eignet sich insbesondere zum Einsatz in Verfahren, bei denen unterschiedliche Temperaturen erwünscht sind. Dies ist beispielsweise bei der Amplifikation von Nukleinsäuren der Fall. Daher sind die am Rotor oder an der Mantelinnenfläche immobilisierten Biomoleküle vorzugsweise Nukleinsäuren, wie beispielsweise Primer, Sonden und dgl., oder thermostabile Enzyme.

Die Temperierelemente innerhalb des Temperierblocks können unterschiedlich angeordnet sein. Nachteilig ist eine Anordnung, die nur aus einem Temperierelement besteht, welches das Heizen und Kühlen des gesamten Behältnisses (mobile und stationäre Phase) ermöglicht. Dies ermöglicht zwar den Einsatz in der erfindungsgemäßen Vorrichtung mit Arrays als Multiplexsystem, hat aber den Nachteil, dass die Heiz- und Kühlraten aufgrund des relativ großen Volumens innerhalb des Behältnisses relativ langsam sind. Die Präzision ist dabei ebenfalls nur gering, da die Heiz- und Kühlelemente immer in gewissem Ausmaß überschwingen müssen. Um die Heiz- und Kühlleistung zu erhöhen, werden die Thermoelemente auf zwei Bereiche aufgeteilt. Dies erlaubt den Einsatz von zwei oder mehreren Elementen bzw. zwei oder mehreren Peltier-Elementen, wobei es von Vorteil ist, die beiden Thermoelemente mit unterschiedlichen Temperaturen zu verwenden.

Überraschenderweise können damit innerhalb des Inkubationsvolumens (mobile Phase) konstant mindestens zwei unterschiedliche Temperaturzonen generiert werden. Sogar bei langsamer Drehung eines innerhalb des Behältnisses befindlichen Zylinders können die Temperaturbereiche aufrechterhalten werden. Dies bedeutet, dass z.B. auch die an der Rotor- bzw. der Behältnis-Oberfläche befindlichen Primer (die ja in die mobile Phase hineinreichen) diese Temperaturunterschiede mitmachen. Dies bedeutet, dass bei zwei konstanten Temperaturbereichen ein Thermoprofil (Thermocycles) für die Primer und somit für die einzelnen Nachweisreaktionen an der mobilen Phase erreicht werden kann. Diese Anordnung ermöglicht insbesondere den Einsatz von mehreren Tausend Primern (Nachweisreaktionen pro Spot) in einem Test, ausgestattet mit einem konstanten Heiz- und Kühlsystem für zwei definierte Temperaturbereiche, womit eine schnelle und präzise Nachweisreaktion erfolgt. Dabei bleibt der Testaufbau über die gesamte Analyse hin intakt (geschlossener Reaktionsraum), die Reaktionen laufen konstant ab und werden nicht unterbrochen (öffnen des Behältnises) und können darüber hinaus noch in Echtzeit vermessen werden.

Die Temperierelemente im Temperierblock sind unabhängig voneinander vorzugsweise als Kühlelemente oder Heizelemente vorgesehen. Die Temperierelemente können sowohl zum Heizen als auch zum Kühlen eingesetzt werden. Die Steuerung kann dabei beispielsweise über einen Rechner oder eine Steuereinheit erfolgen. Im Zuge der Verwendung der erfindungsgemäßen Vorrichtung können die Temperierelemente sowohl ausschließlich als Kühl- oder ausschließlich als Heizelemente verwendet werden. Alternativ dazu können die Temperierelemente wechselnde Funktion (Heiz- oder Kühlelement) erfüllen.

Gemäß einer bevorzugten Ausführungsform der vorliegenden Erfindung sind die Temperierelemente Peltier-Elemente. Weiters geeignet sind auch elektrische Widerstände (elektrische Widerstandsheizungen), mit Flüssigkeiten oder Gasen gefüllte Elemente (Flüssigkeitselemente (Schläuche...) oder Gasstromheizungen), sowie Strahlungsheizungen (Infrarotstrahlung, Mikrowelle (Infrarotstrahler, Mikrowellensender oder Lichtemitter) etc.).

Um die Genauigkeit der Temperatursteuerung an den Temperierelementen nicht oder nur unwesentlich zu beeinflussen, ist zwischen den Temperierelementen des mindestens einen Temperierblocks vorzugsweise ein Zwischenraum oder Isoliermaterial vorgesehen. Im der einfachsten Ausführung ist dies Luft. Es können auch geschäumte Kunststoffe, Glas oder keramische Stoffe verwendet werden.

Ein weiterer Aspekt der vorliegenden Erfindung betrifft die Verwendung einer erfindungsgemäßen Vorrichtung zur Amplifikation von Nukleinsäuren (z.B. PCR). Dabei werden Primer vorzugsweise direkt oder über einen chemischen Linker an die Mantelinnenfläche oder Grundfläche eines Behältnisses gebunden. Bei einer derartigen Ausführungsform kann entweder das Behältnis in der Temperiervorrichtung radial bewegt werden, oder, wenn dieses radial fixiert ist, die Temperierelemente abwechselnd (den PCR-Zyklen angepasst) zum Kühlen bzw. Heizen auf die benötigten Temperaturen temperiert werden. Gemäß einer besonders bevorzugten Ausführungsform der vorliegenden Erfindung werden die Primer an die Oberfläche (Mantelfläche) eines in das Behältnis einbringbaren Rotors gebunden. In dieser Ausführungsform ist das Behältnis radial fixiert. Vorzugsweise werden dabei Primer unterschiedlicher Art an den Oberflächen des Rotors immobilisiert, um eine Vielzahl an Amplifikationen verschiedener Nukleinsäuren parallel durchzuführen.

Mit der vorliegenden Erfindung ist es insbesondere möglich, Nukleinsäureamplifikationen durchzuführen. Dabei kann die erfindungsgemäße Vorrichtung derart ausgestaltet sein, dass diese in der Lage ist, ein Behältnis aufzunehmen, das selbst geeignet ist, einen rotationssymmetrischen Zylinder (Rotor), an dessen Mantelfläche Spots (Nukleinsäuren, Primer) immobilisiert sind, aufzunehmen. Zwischen dem eingesetzten Zylinder und der Innenwand des Behältnisses ist ein Ringspalt vorgesehen, der geeignet ist, Flüssigkeiten (wie z.B. PCR-Lösungen usw.) aufzunehmen. Während der Rotation des Zylinders im Behältnis durchlaufen die am Zylinder gebundenen Nukleinsäuremoleküle in der umgebenden Flüssigkeit ein Temperaturprofil, welches durch die Temperierelemente der erfindungsgemäßen Vorrichtung vorgegeben wird. Die Anzahl der Thermozonen und die Temperaturen sind dabei beliebig einstellbar, da jedes Temperierelement einzeln steuerbar sein kann. Bei diesem Vorgang wird die stationäre Phase (immobilisierte Spots) hinsichtlich der Temperatur zur mobilen Phase. Dies ist auch der wesentliche Unterschied zu Mikrofluidik, bei der Flüssigkeiten durch Kanäle geschickt werden und dabei Zonen unterschiedlicher Temperaturen passieren. Die Nachteile dabei sind, dass alle Reaktionsbestandteile in der mobilen Phase mitgeführt werden müssen. Eine Reaktion an der stationären Phase ist damit unmöglich, das Verwenden von mehreren parallelen Reaktionsnachweisen somit nicht gegeben.

Vorzugsweise ist das Behältnis und/oder der Rotor der erfindungsgemäßen Vorrichtung in getrennte Kammern oder Areale unterteilt.

Die Verwendung von vielen parallelen Reaktionsnachweisen ist durch die Immobilisierung mehrerer gleicher oder unterschiedlicher Reaktionspartner in definierten Reaktionsarealen auf einer Festphase realisierbar (Primerextension EP 0 972 081). Werden die immobilisierten Reaktionspartner mit einer mobilen Phase (Flüssigkeit) überschichtet, die alle generellen Reaktionspartner und die Probenbestandteile enthält, so können die Flüssigkeit und die hineinreichenden immobilisierten Primer temperiert werden. In der erfindungsgemäßen Ausführung erfolgt die Temperierung durch einen Transfer der festen Phase (immobilisierte Primer) durch unterschiedliche Temperaturzonen. Die Temperaturzonen werden über die feste Begrenzung (Behältnis oder allg. Deckel) des Raums für die mobile Phase festgelegt. In einer speziellen Ausführung ist dies das Behältnis, an dem Temperierelemente anliegen, die über ihre Kontaktfläche die Temperaturzone innerhalb des Behältnisses definieren.

Durch die Rotationsbewegung des im Behältnis befindlichen Rotors oder des Behältnisses selbst, durchwandern die immobilisierten Primer die unterschiedlichen Temperaturbereiche, die von den Temperierelementen vorgegeben werden. Dabei weisen die Temperierelemente vorzugsweise jene Temperaturen auf, die für solche Verfahren üblich sind. Die Heiz- und Kühlzeiten werden dabei um ein Vielfaches verkürzt (von 60sec auf 5sec) und ein überschwingen gänzlich verhindert, da die Thermoelemente eine für sich konstante Temperatur aufweisen.

Weiters kann die erfindungsgemäße Vorrichtung zur Durchführung von Enzymreaktionen, wie z.B. DNA-Polymerase-Reaktionen ("solid phase amplification", "arrayed primer extension" (APEX)), DNA-Ligase-Reaktionen, DNA-Methyltransferasen-Reaktionen, Restriktionsendo- und -exonuklease-Reaktionen, Oxidoreduktase-, Hydrolase-, Ligase-, Lyase-, Isomerase-, Phosphatase-, Kinase-, Methylase- und Transferase-R verwendet werden.

Viele unterschiedliche Nachweisreaktionen, wie z.B. jene die auf der Bindung von Primern an DNA/RNA beruhen, können mit der erfindungsgemäßen Vorrichtung besonders zweckdienlich durchgeführt werden. Einige der verschiedenen Primer, die auf der Oberfläche immobilisiert wurden, werden dann gegebenenfalls mit Teilen einer biologischen Probe (Blutextrakt) eine Reaktion auslösen. Diese Reaktion sollte dann in einer vergleichbaren Weise zu einer einzeln durchgeführten Nachweisreaktion ablaufen und somit nicht von den anderen vorkommenden und ebenfalls immobilisierten Primern beeinflusst werden. Dadurch wäre die Parallelität des Testsystems gegeben. Eine Aufteilung der Probe für Einzelnachweise ist damit überflüssig, Aufwand und Kosten werden eingespart. Die Präzision, auf Grund der Vermeindung des statistischen Fehlers bei Aliquotierungen, steigt.

Ein schnelles und genaues Heiz- und Kühlsystem verliert keine Zeit mit Temperaturwechsel der Heiz-/Kühlelemente und unterliegt keinem Fehler während der Zieltemperaturannäherung (Einpendeln). Ist die Heiz- und Kühlzeit auf die Temperierung der Probenflüssigkeit begrenzt, ist die Analysenzeit optimiert. Ein über- oder unterschreiten der Zieltemperaturen ist durch konstante Temperaturzonen verhindert. Fehler durch unbeabsichtigte Nebenreaktionen sind somit stark reduziert und die Zahl an Falsch positiven bzw. falsch negativen Resultaten sinkt.

Die erfindungsgemäße Vorrichtung eignet sich besonders gut für ein Verfahren zur Amplifikation zumindest einer Nukleinsäure in einer Probe umfassend die Schritte:
a) Bereitstellen eines festen Trägers umfassend zumindest zwei daran immobilisierte Primer bestehend aus jeweils zumindest zwei Teilsequenzen, wobei eine erste Teilsequenz am 3'-Ende eine zur amplifizierenden Nukleinsäure komplementäre Nukleotidsequenz aufweist und zumindest eine zweite am 5'-Ende liegende konstante Teilsequenz aufweist, die eine Identität zu einem Teilabschnitt der zu amplifizierenden Nukleinsäure von weniger als 90% aufweist, wobei der Primer über das 5'-Ende der zweiten Teilsequenz am festen Träger immobilisiert ist und wobei die zur amplifizierenden Nukleinsäure komplementären Nukleotidsequenzen von mindestens zwei am Träger immobilisierten Primern als Vorwärts- und Rückwärts-Primer geeignet sind, die zu amplifizierende Nukleinsäure zu amplifizieren,
b) Kontaktieren des festen Trägers mit einer Lösung umfassend eine Probe und mindestens einen Primer, wobei der zumindest eine Primer im Wesentlichen ident mit der konstanten Teilsequenz der immobilisierten Primer und/oder mit den mindestens zwei an festen Trägern immobilisierten Primern ist,
c) Durchführen einer Amplifikationsreaktion zur Herstellung von zumindest einem löslichen Amplifikationsprodukt und
d) gegebenenfalls Detektieren eines am festen Träger über den zumindest einen immobilisierten Primer gebundenen Amplifikationsprodukts.

Derartige Verfahren sind beispielsweise in Pernov A et al. (Nucleic Acid Res. (2005) 33: e11) beschrieben.

In einem ersten Amplifikationszyklus kann dabei gegebenenfalls mindestens eine der ersten Teilsequenzen (spezifische Sequenz) der immoblisierten Primer ein Amplifikationsprodukt aufgrund der Proben-Nukleotidsequenz erzeugen. Das erste Amplifikationsprodukt (Verlängerung des immobilisierten Primers) dient danach als Vorlage für die Amplifikation (Verlängerung) eines zweiten immobilisierten Primers, der sich in räumlicher Nähe (< 1µm) befindet. Dadurch bildet sich gegebenenfalls ein Amplifikationsprodukt zwischen zwei immobilisierten Primern aus. Führt der erste Amplifikationszyklus zu einem Amplifikat, dann kann ein zweiter Amplifikationszyklus durch die in Lösung befindlichen konstanten Primer starten. Dabei dient die im immobilisierten Amplifikat enthaltene zu der Sequenz des 5'Endes der immobilisierten Primer komplementäre Sequenz als Vorlage für die konstanten löslichen Primer. Das gegebenenfalls dadurch erzeugte Amplifikat ist nun löslich. In einem dritten Amplifikationszyklus werden die durch Zyklus eins und zwei erzeugten löslichen Amplifikate bei jedem weiteren Zyklus durch die konstanten Primer in einer löslichen PCR Reaktion amplifiziert. Da alle Amplifikate aus der Festphasenreaktion in Lösung durch die selben Primer amplifiziert werden, ist die Amplifikationsrate für alle Zielsequenzen nahezu ident. Das Annealing erfolgt unter Bedingungen, die es ermöglichen, dass die Primer spezifisch an deren Target-Sequenzen binden können. Entsprechend sind die Primer designed. Hierfür gibt es Verfahren, die dem Fachmann hinreichend bekannt sind.

Mit dem Verfahren der oben beschriebenen Art wird die Signalgenerierung über drei verschiedene Amplifikationszyklen (Festphasen Primer Extension, Festphasen PCR und konstante Flüssigphasen PCR) erreicht. Mit dem erfindungsgemäßen Verfahren ist es zudem möglich Multiplex PCR spezifisch und sensitiv durchzuführen, da einerseits die Anzahl der in Lösung befindlichen Primer signifikant reduziert werden kann, da die immobilisierten Primer mit der konstanten Nukleotidsequenz gemeinsam mit einem Sequenz-spezifischen Primeranteil zur Amplifikation sämtlicher Nukleinsäuren geeignet sind, und andererseits die Amplifikation in Lösung die erforderliche Sensitivität erreicht (exponentielle Amplifikation). Das erfindungsgemäße Verfahren eignet sich zudem gleichermaßen gut zum Nachweis von DNA und RNA in einer Probe.

Die Signalgenerierung (d.h. die Nachweisreaktion) kann dann über die sequenzspezifische Bindung der Amplifkiationsprodukte an die immobilisierten Primer am festen Träger erfolgen, wie z.B. durch spezifische DNA Interkalatoren (SYBR Green I, Eva Green...), durch zuvor in das Amplifikat eingebaute Farbstoffe oder andere spezifische Reaktionen (Farbreaktionen, chemische oder physikalsiche Nachweisreaktionen), die das Vorhandensein von Amplifikaten anzeigen.

Der Vorteil der Methode liegt einerseits in der spezifischen initialen Nachweisreaktion und der hohen Sensitivität durch die exponentielle Vermehrung in Flüssigkeit und andererseits in der hochparallelen Anwendung einer großen Anzahl von Primern (> 10 Spots) am festen Träger pro cm².

Ein weiterer Vorteil des erfindungsgemäßen Verfahrens besteht darin, dass aufgrund der unterschiedlichen Amplifikate unterschiedliche Schmelztemperaturen zu den immobilisierten Primern bzw. Amplifikaten gegeben sind. Dadurch lässt sich in einem weiteren Zyklus zu jedem Spot (immobilisiertes Primerpärchen) eine so genannte Schmelzkurve erzeugen. Dabei werden Messungen der Spots bei steigenden Temperaturen vorgenommen und die Signalhöhen sequenziell aufgezeichnet. Durch sequenzabhängiges Schmelzen der Nukleotiddoppelstränge ändert sich die Signalhöhe, einerseits durch Dissoziation des markierten Amplifikates oder andererseits durch Freisetzung des Doppelstranginterkalators (Farbstoffs z.B. SYBR Green). Die so erhaltene Schmelzkurve kann gegebenenfalls mit Sollwerten verglichen werden. Damit können Rückschlüsse auf die Sequenz des Amplifikats und somit auf die Richtigkeit der Amplifikationsreaktion gezogen werden.

Die vorliegende Erfindung ist weiters durch die folgenden Figuren und Beispiele erläutert, ohne jedoch auf diese beschränkt zu sein.

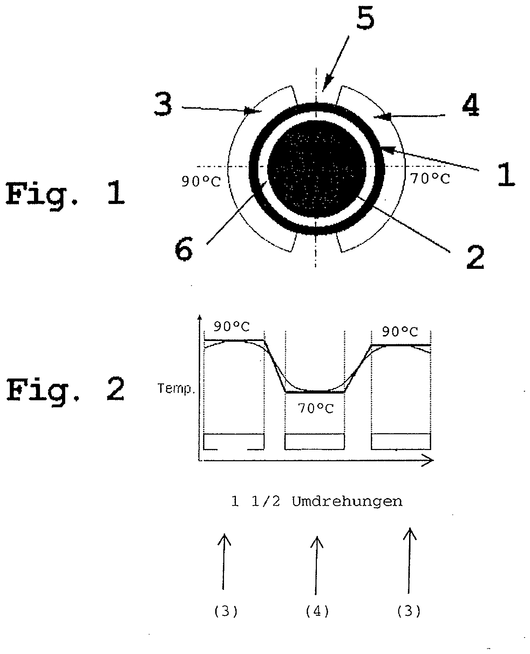

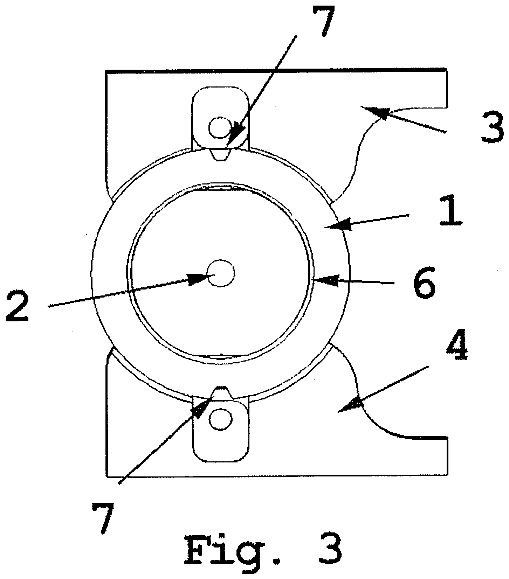

Fig. 1 zeigt eine erfindungsgemäße Vorrichtung, in der ein Behältnis angeordnet ist, Fig. 2 zeigt den Temperaturverlauf an einem definierten Punkt der Mantelfläche des in einem Behältnis befindlichen Rotors innerhalb einer 1½ Umdrehung, Fig. 3 zeigt eine Draufsicht der erfindungsgemäßen Vorrichtung mit einem des angeordneten Behältnis in dem sich ein Rotor befindet.
Fig. 1 zeigt eine Draufsicht eines in einem Behältnis 1 befindlichen Rotors 2, wobei zwischen der Innenseite des Behältnisses 1 und des Rotors 2 ein Ringspalt 6 vorgesehen ist, der mit Flüssigkeit gefüllt sein kann. Die Wärmeaustauschbacken 3, 4 lassen an den Berührungsflächen einen gleich großen Spalt 5 frei. Der Spalt 5 dient einerseits der Isolation (Luft) und als Übergangszone von einer zur anderen Temperatur und andererseits ist der Spalt 5 auch eine Zone, bei der von außerhalb des Behältnisses 1 bzw. der erfindungsgemäßen Vorrichtung Proben bzw. Umsetzreaktionen detektiert werden können. Die Temperaturen im Behältnis 1 (insbesondere im Ringspalt 6 zwischen dem Behältnis 1 und dem Rotor 2) liegen bei einer PCR beispielsweise zwischen 98°C und 4°C. Die Heizbacken 3, 4 liegen flächig an der Behältnisaußenseite an und können ein wenig über- bzw. unterragen. Das Material kann eine Aluminiummischung, Stahl, Kupfer oder ein anderes, gut wärmeleitfähiges Material sein (Heiz- und Kühlfolien, flüssigkeitsgefüllte, starre oder flexible Behälter bzw. Säcke). An der Rückseite der Backen 3, 4 ist ein Heizelement, wie z.B. ein Peltier-Element, angebracht. Dies könnte alternativ z.B. durch eine elektrische Widerstandsheizung, Wärmestrahlung, Ultraschall, Mikrowelle usw. ersetzt werden.
Fig. 2 zeigt den theoretischen Verlauf der Flüssigkeitstemperatur und den experimentellen Verlauf. Aus der Verlaufskurve wird deutlich, dass beide eingestellten Temperaturen erreicht werden. Die Steigung der Kurve zwischen den Temperaturen zieht sich etwas in die Heizzonen hinein, was durch Wärmediffusion in der Flüssigkeit, aber auch innerhalb des Behältnismaterials bedingt ist. Zusätzlich kommt es während der Bewegung zur leichten Durchmissung der Flüssigkeiten wodurch eine scharfe Temperaturgrenze verhindert wird. Offensichtlich ist, dass die Voraussetzungen für eine Solid Phase Amplifikation (bzw. OnSpot PCR) gegeben sind, da die über der stationären Phase befindliche Flüssigkeit, und somit auch die in diese Flüssigkeit reichenden immobilisierten Moleküle (Primer), die geforderten Temperaturstufen erreicht.
Fig. 3 zeigt eine Draufsicht der erfindungsgemäßen Vorrichtung. Die Wärmeaustauschbacken 3 und 4'umschließen unter Freilassung eines Spalts ein rotationssymmetrisches Behältnis 1, in dem sich ein Rotor 2 befindet. Durch eine Arretierungsvorrichtung 7, die an einer oder beiden der Wärmeaustauschbacken 3 und 4 angeordnet sein kann, wird der in der Vorrichtung positionierte Behälter 1 translatorisch und/oder rotatorisch fixiert. Dadurch wird verhindert, dass bei sich bewegendem Rotor 2 das Behältnis 1 mitbewegt wird.

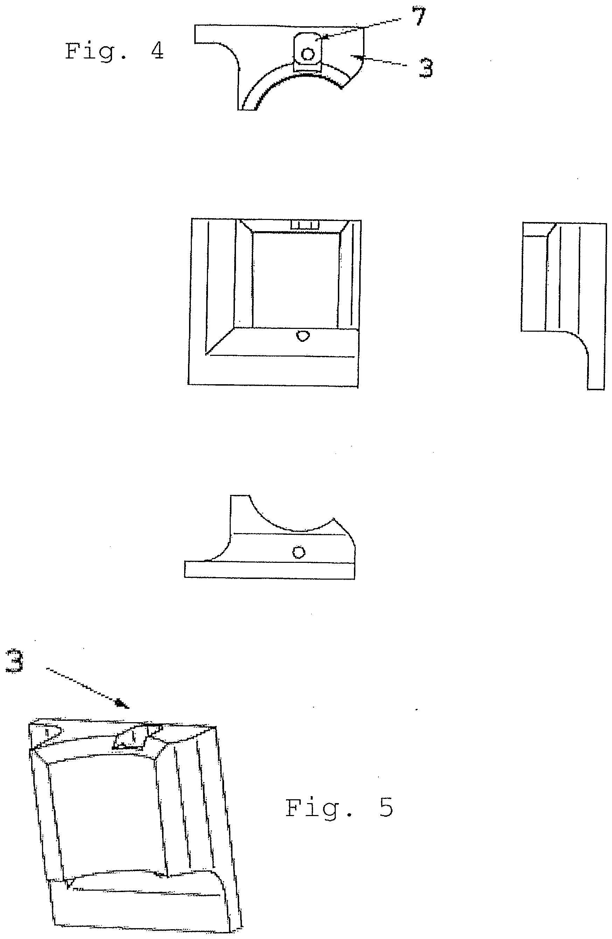

Fig. 4 zeigt eine Draufsicht einer erfindungsgemäßen Wärmeaustauschbacke 3, an der eine Arretierungsvorrichtung 7 vorgesehen ist.
Fig. 5 zeigt eine dreidimensionale Ansicht der erfindungsgemäßen Wärmeaustauschbacken 3.

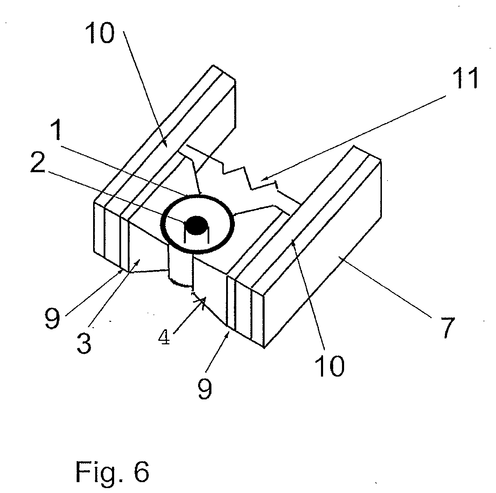

Fig. 6 zeigt eine dreidimensionale Ansicht einer erfindungsgemäßen Vorrichtung 7. Die Vorrichtung 7 umfasst einen Temperierblock, der zwei Wärmeaustauschbacken 3, 4 aufweist. An der vom aufnehmenden Behältnis 1 abgewandten Seite der Wärmeaustauschbacken 3 ist je ein Temperierelement 9 (z.B. Pelierelement) angeordnet. Die Wärmeaustauschbacken 3, 4 sind jeweils an einem Stützelement 10 befestigt, welche über eine Zugvorrichtung 11 (z.B. Faden) miteinander verbunden sind. Dadurch wird ermöglicht, dass die Backen 3, 4 genügend an das Behältnis 1 angepresst werden, um den nötigen Temperaturzustand mit demselben zu ermöglichen.

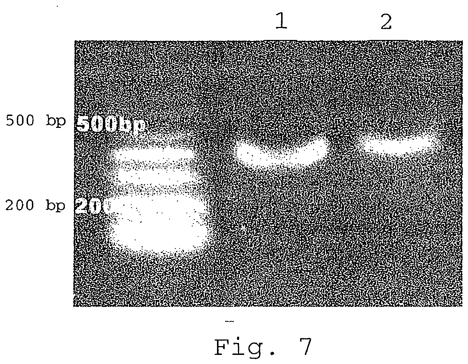

Fig. 7 zeigt das Ergebnis einer Flüssigphasen-PCR (Bahn 1) mit einem in der hybcell mit unterschiedlich angesteuerten Heizbacken durchgeführten PCR (Bahn 2).

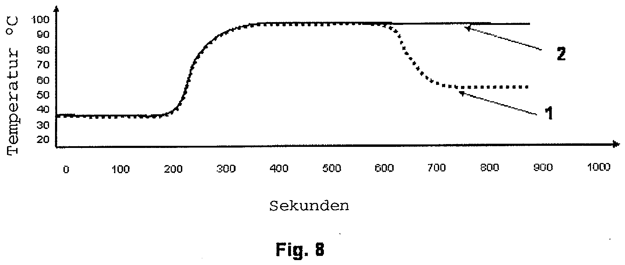

Fig. 8 zeigt die Temperaturen der Backen während der ersten 1000 sec.

### BEISPIELE:

### Beispiel 1:

### Gegenüberstellung von bekannter Solid Phase Amplifikation (SPA) und der gekoppelten SPA/PCR (OnSpot PCR).

Die Detektion von humanen pathogenen Erregern (vorwiegend Bakterien) in Blutproben ist eine wichtiges Betätigungsfeld der molekularen DNA Diagnostik. Eingesetzt werden PCR, Realtime PCR und SPA. Die SPA wird vorwiegend als Einzelreaktion in kleinen Gefäßen oder Näpfchen von Multititerplatten durchgeführt. Um eine große Anzahl an verschiedener Pathogenen nachzuweisen ist die Anwendung der SPA als Multiplex Reaktion in Array Format sinnvoll. Die Probe muss nicht in viele Einzelreaktionen aufgeteilt werden und die Kosten für Reagenzien steigen zur Nachweiszahl unproportional flach.

Es wurden Primerpärchen für 2 verschiedene Bakterien designed.

Die gleichen Primer wurden ein zweites Mal mit dem Unterschied synthetisiert, dass sie am 5'-Ende eine für alle konstante Sequenz zusätzlich enthielten.

Die verwendete Feste Phase (Träger) war in allen Tests der gleiche. Ein zylindrischer Körper (hybcell) überzogen mit einer Silliziumoxid Schicht. Die Bindung der Oligos erfolgte über eine Aldehyd-Amino Gruppen Reaktion. Die Herstellung der Spots an der Oberfläche wurde durch einen Piezodrucker der Firma Scienion bewerkstelligt.

Danach wurde für beide Systeme der gleiche Reaktionsmix zusammengestellt, beinhaltend: die Bestandteile für eine PCR Reaktion, fluoreszenz markierte Nukelotide, eine definierte Menge zur amplifizierender DNA (Target). Der OnSpot Reaktion wurde noch eine definierte Menge des konstanten Primerpärchens zugesetzt. Die Amplifikationreaktion erfolgte durch Zyklen von Heizen und Kühlen. Bestimmt wurden die nötige Anzahl an Zyklen um ein Signal-Rauschverhältnis von > 6 zu generieren.

**Tabelle: SPA versus OnSpot PCR**

| **Zyklenanzahl** | **1** | **10** | **20** | **30** | **40** | **50** | **60** |
|---|---|---|---|---|---|---|---|
| ASPA | 0,25 | 0,26 | 0,25 | 0,29 | 0,28 | 0,41 | 0,51 |
| BSPA | 0,23 | 0,21 | 0,24 | 0,23 | 0,24 | 0,35 | 0,46 |
| A OnSpot | 0,24 | 0,25 | 0,29 | 1,45 | 7,25 | 25,00 | 56,23 |
| B OnSpot | 0,16 | 0,15 | 0,27 | 1,35 | 6,75 | 21,38 | 48,98 |

### Legende:

Zyklenzahl: Messpunkt mit der Anzahl an geleisteten Amplifikationszyklen
A SPA; B SPA: Testserie der Solid Phase Amplifikation für Bakterium A (Staph. aureus) und B (Enterococcus aerogenes)
A OnSpot; B OnSpot: Testserie der gekoppelte OnSpot PCR für Bakterium A (Staph. aureus) und B (Enterococcus aerogenes)
Ergebniszahlen: entsprechen den erhaltenen Verhältnissen aus Spotsignal zu Hintergrundsignal.

### Beispiel 2: Flüssigphasen-PCR mit Primer umfassend eine konstante Teilsequenz und eine Target-spezifische Teilsequenz.

Für die Durchführung der Flüssigphasen-PCR wurden dieselben Primer eingesetzt, die in Beispiel 1 (OnSpot PCR) an einem festen Träger immobilisiert wurden. Der Unterschied besteht darin, dass alle Primer bei dieser Variante in Lösung vorliegen. Ziel ist dabei der parallele Einsatz von vielen Primerpaaren in einer Testlösung ohne gegenseitige Beeinflussung (unspezifischen Amplifikation). Gewährleistet wird dies durch eine sehr geringe Konzentration der Primer mit der Target-spezifischen Teilsequenzen und eine hohe Konzentration an Primern mit der konstanten Teilsequenz (gleich den gelösten Primern in der OnSpot PCR, siehe Beispiel 1). Die so erhaltenen unterschiedlichen Amplifikate sind in einem zweiten Schritt zu identifizieren, z.B. Hybridisierung an immobilisierten Sonden (Array) oder Größenauftrennung im Gel.

Während der PCR wurden die Backen in der hybcell mit unterschiedlichen Temperaturen angesteuert (120 °C und 60 °C). Dies fürhte ebenfalls zu einer PCR (vgl. Fig. 7 und 8).

Dabei zeigt Fig. 7 ein Agarosegel mit aufgetrennten DNA Fragmenten. Die Fragmente wurden mit SYBR Green gefärbt. Am linken Rand ist ein Standard zu sehen. Er gibt die ungefähre Größe der Fragmente wieder. Bahn 1 zeigt ein DNA Fragment (Amplicon) aus einer Flüssigphasen PCR nach 30 Temperaturzyklen. Bahn 2 zeigt ein Amplicon, das in der hybcell bei unterschiedlich angesteuerten Heizbacken und Rotation des Zylinders erzeugt wurde. Vorgeschalten war ein Temperaturschritt zur Aktivierung der Polymerase (Standardschritt) und dann wurde die Temperatur der Backen unterschiedlich angesteuert. Die Rotationsgeschwindigkeit des Zylinders war mit 1 rpm festgelegt. Dh. die Verweilzeit der Spots bzw. der umgewältzten Flüssigkeit in den beiden Temperaturbereichen war ca. 20 sec.

Fig. 8 zeigt die aufgezeichnete Temperatur der Backen während der ersten 1000 sec. 1 ist die Temp. der einen Backe und 2 die der anderen Backe.

## Patentansprüche

1. Vorrichtung umfassend ein rotationssymmetrisches Behältnis (1) mit einer Mantelfläche und/oder einer Grundfläche, wobei das Behältnis (1) einen einsetzbaren rotationssymmetrischen Rotor (2) aufweist, wobei zwischen dem Behältnis (1) und dem Rotor (2) ein Ringspalt (6) vorgesehen ist und der Rotor (2) mindestens einen Strömungskanal zur Beförderung von Flüssigkeiten und/oder Gasen in und/oder aus dem Innenraum des Behältnisses (1) aufweist, an der Oberfläche des Rotors (2) oder an der Mantelinnenfläche des Behältnisses (1) organische Moleküle immobilisiert sind, **dadurch gekennzeichnet, dass** die Vorrichtung einen zur Aufnahme des Behältnisses (1) geeigneten Temperierblock mit mindestens zwei Temperierelementen (3, 4) umfasst, wobei die Temperierelemente (3, 4) des mindestens einen Temperierblocks mit der Mantelfläche des zu temperierenden Behältnisses (1) in Wärmeaustauschkontakt ist und der Temperierblock eine dem Behältnis äquivalente Form aufweist und die Temperierelemente (3, 4) zur Generierung unterschiedlicher Temperaturzonen angeordnet sind, wobei im Zuge einer Rotation eine Oberfläche des Rotors (2) den unterschiedlichen Temperaturzonen ausgesetzt ist.

2. Vorrichtung nach Anspruch 1, **dadurch gekennzeichnet, dass** das Behältnis (1) translatorisch relativ zum mindestens einen Temperierblock bewegbar ist.

3. Vorrichtung nach Anspruch 1 oder 2, **dadurch gekennzeichnet, dass** das rotationssymmetrische Behältnis (1) die Form eines Zylinders aufweist.

4. Vorrichtung nach einem der Ansprüche 1 bis 3, **dadurch gekennzeichnet, dass** an der Oberfläche des Rotors (2) organische Moleküle immobilisiert sind.

5. Vorrichtung nach einem der Ansprüche 1 bis 4, **dadurch gekennzeichnet, dass** an der den Temperierelementen gegenüberliegenden Oberfläche der Grundfläche des Behältnisses (1) organische Moleküle immobilisiert sind.

6. Vorrichtung nach einem der Ansprüche 1 bis 4, **dadurch gekennzeichnet, dass** an der Mantelinnenfläche des Behältnisses (1) organische Moleküle immobilisiert sind.

7. Vorrichtung nach einem der Ansprüche 4 bis 6, **dadurch gekennzeichnet, dass** die organische Moleküle Nukleinsäuren oder Enzyme sind.

8. Vorrichtung nach einem der Ansprüche 1 bis 7, **dadurch gekennzeichnet, dass** die Temperierelemente (3, 4) unabhängig voneinander als Kühlelemente oder Heizelemente vorgesehen sind.

9. Vorrichtung nach einem der Ansprüche 1 bis 8, **dadurch gekennzeichnet, dass** die Temperierelemente (3, 4) vorzugsweise Peltier-Elemente, elektrische Widerstände, Infrarotstrahler, Mikrowellensender, mit Flüssigkeit oder Gasen gefüllte Elemente, Lichtemitter oder Kombinationen davon sind.

10. Vorrichtung nach einem der Ansprüche 1 bis 9, **dadurch gekennzeichnet, dass** das Behältnis (1) in Wärmeaustauschkontakt mit den Temperierelementen (3, 4) relativ zum Temperierblock fixiert ist.

11. Vorrichtung nach einem der Ansprüche 1 bis 10, **dadurch gekennzeichnet, dass** zwischen den Temperierelementen des mindestens einen Temperierblocks ein Zwischenraum vorgesehen ist.

12. Vorrichtung nach einem der Ansprüche 1 bis 11, wobei das Behältnis und/oder der Rotor in getrennte Kammern oder Areale unterteilt ist.

13. Verwendung einer Vorrichtung nach einem der Ansprüche 1 bis 12 zum Heizen und Kühlen von flüssigen oder gasförmigen biologischen Proben.

14. Verwendung einer Vorrichtung nach einem der Ansprüche 1 bis 12 zum Nachweis von Nukleinsäuren.

## Claims

1. A device comprising a rotationally symmetrical container (1) with a lateral area and/or a base area, wherein the container (1) has an insertable rotationally symmetrical rotor (2), wherein an annular gap (6) is provided between the container (1) and the rotor (2) and the rotor (2) has at least one flow channel for conveying liquids and/or gases into and/or out of the interior of the container (1), and wherein organic molecules are immobilized on the surface of the rotor (2) or on the inner lateral area of the container (1), **characterized in that** the device comprises a tempering block, for accommodating the container (1) having at least two tempering elements (3, 4), wherein the tempering elements (3, 4) of the at least one tempering block are in heat-exchanging contact with the lateral area of the container (1) to be thermally regulated and the shape of the tempering block is equivalent to the container, and the tempering elements (3, 4) are arranged for generating different temperature zones, wherein a surface of the rotor (2) is exposed to the different temperature zones during the course of a rotation.

2. The device according to claim 1, **characterized in that** the container (1) can be moved in a translatory fashion relative to the at least one tempering block.

3. The device according to claim 1 or 2, **characterized in that** the rotationally symmetrical container (1) has the shape of a cylinder.

4. The device according to one of claims 1 to 3, **characterized in that** organic molecules are immobilized on the surface of the rotor (2).

5. The device according to one of claims 1 to 4, **characterized in that** organic molecules are immobilized on the surface of the base area of the container (1), opposite to the tempering elements.

6. The device according to one of claims 1 to 4, **characterized in that** organic molecules are immobilized on the inner lateral area of the container (1).

7. The device according to one of claims 4 to 6, **characterized in that** the organic molecules are nucleic acids or enzymes.

8. The device according to one of claims 1 to 7, **characterized in that** the tempering elements (3, 4) are provided as independent cooling elements or heating elements.

9. The device according to one of claims 1 to 8, **characterized in that** the tempering elements (3, 4) are preferably Peltier elements, electrical resistors, infrared radiators, microwave transmitters, elements filled with liquids or gases, light emitters or combinations thereof.

10. The device according to one of claims 1 to 9, **characterized in that** the container (1) is fixed relative to the tempering block in heat-exchanging contact with the tempering elements (3, 4).

11. The device according to one of claims 1 to 10, **characterized in that** a space is provided between the tempering elements of the at least one tempering block.

12. The device according to one of claims 1 to 11, **characterized in that** the container and/or the rotor is divided into separate chambers or areas.

13. Use of a device according to one of claims 1 to 12 for heating and cooling liquid or gaseous biological samples.

14. Use of a device according to one of claims 1 to 12 for detecting nucleic acids.

## Revendications

1. Dispositif comprenant un contenant symétrique en rotation (1) avec une surface d'enveloppe et/ou une surface de base, le contenant (1) comportant un rotor (2) symétrique en rotation pouvant être mis en oeuvre, un espace annulaire (6) étant prévu entre le contenant (1) et le rotor (2) et le rotor (2) comportant au moins un conduit d'écoulement pour le transport de liquides et/ou de gaz dans et/ou à partir de l'espace intérieur du contenant (1), des molécules organiques étant immobilisées à la surface du rotor (2) ou à la surface intérieure d'enveloppe du contenant (1), **caractérisé en ce que** le dispositif comprend un bloc de régulation de température adapté pour loger le contenant (1), avec au moins deux éléments de régulation de température (3, 4), les éléments de régulation de température (3, 4) d'au moins un bloc de régulation de température étant en contact d'échange thermique avec la surface d'enveloppe du contenant (1) à réguler en température et le bloc de régulation de température comportant une forme équivalente au contenant et les éléments de régulation de température (3, 4) étant disposés pour générer des zones de température différentes, une surface du rotor (2) étant au cours de rotation exposée aux différentes zones de température.

2. Dispositif selon la revendication 1, **caractérisé en ce que** le contenant (1) peut être mobile en translation par rapport à au moins un bloc de régulation de température.

3. Dispositif selon la revendication 1 ou 2, **caractérisé en ce que** le contenant (1) symétrique en rotation comporte la forme d'un cylindre.

4. Dispositif selon l'une quelconque des revendications 1 à 3, **caractérisé en ce que** des molécules organiques sont immobilisées à la surface du rotor (2).

5. Dispositif selon l'une quelconque des revendications 1 à 4, **caractérisé en ce que** des molécules organiques sont immobilisées sur la surface de la surface de base du contenant (1), opposée aux éléments de régulation de température.

6. Dispositif selon l'une quelconque des revendications 1 à 4, **caractérisé en ce que** des molécules organiques sont immobilisées sur la surface intérieure d'enveloppe du contenant (1).

7. Dispositif selon l'une quelconque des revendications 4 à 6, **caractérisé en ce que** les molécules organiques sont des acides nucléiques ou des enzymes.

8. Dispositif selon l'une quelconque des revendications 1 à 7, **caractérisé en ce que** les éléments de régulation de température (3, 4) sont prévus indépendamment l'un de l'autre sous forme d'éléments de refroidissement ou d'éléments de chauffage.

9. Dispositif selon l'une quelconque des revendications 1 à 8, **caractérisé en ce que** les éléments de régulation de température (3, 4) sont de préférence des éléments Peltier, des résistances électriques, des émetteurs de rayons infrarouges, des émetteurs de micro-ondes, avec des éléments remplis de liquide ou de gaz, des émetteurs de lumière ou des combinaisons de ceux-ci.

10. Dispositif selon l'une quelconque des revendications 1 à 9, **caractérisé en ce que** le contenant (1) est fixé en contact d'échange thermique avec les éléments de régulation de température (3, 4) par rapport au bloc de régulation de température.

11. Dispositif selon l'une quelconque des revendications 1 à 10, **caractérisé en ce qu'**un espace intermédiaire est prévu entre les éléments de régulation de température d'au moins un bloc de régulation de température.

12. Dispositif selon l'une quelconque des revendications 1 à 11, le contenant et/ou le rotor étant subdivisé en chambres ou zones e.

13. Utilisation d'un dispositif selon l'une quelconque des revendications 1 à 12 pour le chauffage ou le refroidissement d'échantillons biologiques liquides ou gazeux.

14. Utilisation d'un dispositif selon l'une quelconque des revendications 1 à 12 pour la détection d'acides nucléiques.
